Europäisches Patentamt

European Patent Office

Office européen des brevets

(19)

(11) EP 1 452 598 A1

(12) **EUROPEAN PATENT APPLICATION**
published in accordance with Art. 158(3) EPC

(43) Date of publication:
01.09.2004 Bulletin 2004/36

(21) Application number: 02770278.6

(22) Date of filing: 28.10.2002

(51) Int Cl.7: **C12N 15/10**, C12Q 1/68,
G01N 33/53, G01N 33/566,
G06F 17/50

(86) International application number:
PCT/JP2002/011163

(87) International publication number:
WO 2003/038091 (08.05.2003 Gazette 2003/19)

(84) Designated Contracting States:
AT BE BG CH CY CZ DE DK EE ES FI FR GB GR
IE IT LI LU MC NL PT SE SK TR
Designated Extension States:
AL LT LV MK RO SI

(30) Priority: 29.10.2001 JP 2001331732

(71) Applicants:
• Japan Science and Technology Agency
Kawaguchi-shi, Saitama 332-0012 (JP)

• National Institute of Advanced Industrial
Science and Technology
Tokyo 100-8921 (JP)

(72) Inventor: ARITA, Masanori
Bunkyo-ku, Tokyo 112-0002 (JP)

(74) Representative: Holliday, Louise Caroline
D Young & Co,
21 New Fetter Lane
London EC4A 1DA (GB)

(54) **OLIGONUCLEOTIDE SEQUENCES FREE FROM MISHYBRIDIZATION AND METHOD OF DESIGNING THE SAME**

(57) The present invention is to provide a method for efficiently and systematically designing DNA sequences that avoid mishybridization to each other. After selecting a template such that its Hamming distance of a fixed value k is kept against its reverse sequence and sequences constructed by shifting or concatenating the sequence and its reverse, a set of DNA sequences of predetermined length is specified by the combination of the selected binary string of 0 and 1 (template), and the codewords of any error correcting code such as the Hamming code. A set of DNA sequences thus represented by the template and the error correcting code of minimum distance k can guarantee at least k mismatches between any of the resulting DNA sequences and their concatenations.

EP 1 452 598 A1

**Description**

**Technical Field**

The present invention relates to a set S of oligonucleotide sequences wherein the set S is designed to prevent mishybridization between any sequence in the set S of oligonucleotide sequences of a certain fixed length and other sequences in the set S including the overlap parts of their concatenated sequences by guaranteeing a certain amount of mismatches, a systematic method for designing the above mentioned set of sequences, a systematic method for designing a GC template or an AG template used for designing the above set S of oligonucleotide sequences, DNA or RNA chips, DNA or RNA tags, DNA or RNA computing systems, and DNA or RNA probes utilizing the set S of oligonucleotide sequences.

**Background Art**

DNAs have a structure wherein four types of base, that is, adenine (A), cytosine (C), guanine (G) and thymine (T), are ligated together like a strand. Since A and T, and C and G form a base pair by hydrogen bond respectively, A-T and C-G are considered to be complementary. Two DNA strands have a complementary double helix structure, and the DNA double helix is separated into single-stranded DNAs when temperature rises, and the single-stranded DNA binds to a complementary strand again when temperature drops. This process of binding to a complementary strand is called hybridization, and it is known that the temperature at which DNA strands separate or hybridize depends on GC content in the sequence.

It is pointed out that there is a problem of interaction between primers in designing two types of primers which are indispensable to conduct PCR (polymerase chain reaction), a very useful gene amplification method and an essential technique in wide range of biology-related studies. As the concentration of primers in PCR reaction liquid is higher than that of a target gene by far, if the primers have a structure prone to hybridize each other, mishybridization will occur between sense strands, antisense strands, or sense strand and antisense strand, and so-called primer dimers are formed, with the result that hybridization with the target gene will be drastically suppressed.

Further, in so-called DNA computing that comprises the steps of; synthesizing DNA sequences in which various symbol manipulation operating systems such as logical expression and graph structure are recorded, and cutting and pasting the sequences according to protocols of biological experiments, DNAs as basic parts are synthesized in the number that corresponds to the size of problems, and the problems are solved by a very simple "generate and test" method (Science 266, 1021-1024, 1994). In other words, DNA computing can be carried out by generating DNA sequences randomly in an amount sufficient to cover solution space by connecting parts randomly, and by extracting only solutions that meet a certain requirement from the numerous combinations of the randomly generated sequences. For example, digestion by restriction enzyme can be used for the extraction of the solution mentioned above, and parts are designed so that sequences of incorrect solution contain a recognition site of a restriction enzyme and that sequences of correct solution do not contain a recognition site of a restriction enzyme. DNA memory wherein 5'-end of DNA is fixed on a solid phase is known as an application of such DNA computing model (Nature 403, 175-179, 2000), and a method for searching solutions by generating various combinations of sequences randomly and fixing them on a solid phase and serially cutting out inappropriate sequences from them, is used. In that method, restriction enzymes are used for cutting out sequences on the solid phase, and polymerase is used for extension. In case of this DNA memory, attention should be directed to prevent mishybridization between DNA sequences.

It is also known to design DNAs wherein mishybridization does not occur between DNA sequences in the above-mentioned primer designing, DNA computing, etc. For instance, a programmed computer system comprising means for designing oligonucleotide sequences based on the GenBank database of DNA and mRNA sequences and performing correct and incorrect match modeling with user-selected gene sequences, and means for performing hybridization strength modeling on gene sequences, etc (Published Japanese Translation of PCT International Publication for Patent Application No. 8-503091); a method for DNA computing by computer using genetic algorithm wherein shift-errors are prevented or minimized in consideration of the Hamming distance in frame shift-error hybridization process in which DNA sequences of fixed length are shifted each other ("A New Metric for DNA Computing" Proceedings of the 2nd Annual Genetic Programming Conference, Palo Alto, 472-478, 1997); a method for DNA computing by computer wherein the method is imposed a condition that subsequences of specific length in DNA sequences of fixed length do not appear more than once in designed DNA sequence sets of fixed length (European Patent Application No. 97302313, US Patent No. 5604097), are also reported.

DNA computing is a study field wherein computing of combinatorial mathematics, logic, etc. is conducted by biological experiments as mentioned above. Specifically, it is computing that comprises the steps of; artificially synthesizing DNA sequences in which various symbol manipulation operating systems such as logical expression and graph structure are recorded, and cutting and pasting the sequences according to protocols of molecular biological experiments, and sequences obtained at the end of the experiments are "calculation results" of DNA computing. Thus, demand of a technique used by encoding information that has artificially created meaning (for example, logical parameter, mathematics, etc.) onto DNA base is thought to be increasing acceleratedly with the progress of biotechnology. In order to make the technique work well, it is indispensable to design DNA sequences skillfully in advance to avoid misinterpretation caused by errors. For instance, in case symbol x is expressed as four bases of ACAC, a string xx would be ACACACAC, and a base sequence of x appears in the joint part, and it causes errors. In order to prevent this, there is a need for a method for systematically and efficiently searching a set of sequences, wherein any sequence contains ligating sites to other sequence or between sequences, and a certain amount of mismatches is guaranteed.

As aforementioned, though methods for designing sequences wherein oligonucleotide sequences are constructed such that oligonucleotide sequences such as DNA sequences induce mismatches and can avoid mishybridization with each other are known, these methods are aimed at design of oligonucleotide such as DNA sequences to be fixed on a solid phase, and therefore, sequences that contain shift and ligation in oligonucleotide sequences to avoid mishybridization are not designed. For instance, a method for designing sequences that ensures that mishybridization is avoided even if DNA sequences are in a liquid phase or sequences are ligated each other, has not been reported so far. Further, conventional sequence design that avoids mishybridization is DNA computing by a computer using genetic algorithm, or a very simple "generate and test" method or a modified method thereof, and these DNA computing methods are not regarded as systematic computing methods.

The object of the present invention is to provide a method for systematically designing a set S of oligonucleotide sequences of predetermined length n (n is an integer, 3 or more, preferably, 6 or more), wherein each of oligonucleotide sequences in the set S induces equal to or more than a fixed, predetermined number of mismatches against any of oligonucleotide sequences in the set S, a complementary sequence of each of oligonucleotide sequences in the set S, sequences constructed by shifting these sequences, and sequences produced by ligation of these oligonucleotide sequences, of their complementary sequences, and of the oligonucleotide sequences and their complementary sequences. The set S of oligonucleotide sequences can avoid mishybridization between them, their complementary sequences, sequences constructed by shifting these sequences, and sequences produced by ligation of the oligonucleotide sequences, of their complementary sequences, and of the oligonucleotide sequences and their complementary sequences. In addition, the object of the present invention is to provide a method for systematically designing a set S of oligonucleotide sequences wherein mishybridization can be avoided for reverse sequences as well as for complementary sequences. Meanwhile, "to be able to avoid mishybridization between oligonucleotide sequences by inducing mismatches of predetermined value or more" is hereinafter sometimes referred to as "to be orthogonal", and "a sequence that is orthogonal" is hereinafter sometimes referred to as "an orthogonal sequence".

The present inventor has conducted intensive study for a systematic sequence design method for orthogonal sequences including shift and ligation, which is an important technique for obtaining correct experimental results in DNA computing and biotechnology in future, and has found that a set S of orthogonal oligonucleotide sequences that ensures a mishybridization value including shift and ligation by: 1) selecting a binary string (GC template) such that its Hamming distance to its reverse sequence, to its block shift, and its Hamming distance to the overlap part of its tandem concatenation, its concatenation with its reverse sequence, and the tandem concatenation of its reverse sequence, is equal to or above the predetermined value k, and in the following, an oligonucleotide sequence of length n is specified by the binary string of 0 and 1 (GC template) of predetermined length L (L is an integer 6 or more), meaning that the positions of G or C ([GC]), or A or T ([AT]) are fixed; 2) combining the codewords of any error correcting code with the selected GC template to specify a set of oligonucleotide sequences that induce at least k mismatches between any of them. The present invention has been thus completed.

## Disclosure of the Invention

The present invention relates to a set S of oligonucleotide sequences of predetermined length n (n is an integer 3 or more), wherein each of oligonucleotide sequences in the set S induces equal to or more than a fixed, predetermined number of mismatches against any of oligonucleotide sequences in the set S, a complementary sequence of each of oligonucleotide sequences in the set S, sequences constructed by shifting these sequences, and sequences produced by ligation of these oligonucleotide sequences, of their complementary sequences, and of the oligonucleotide sequences and their complementary sequences, and wherein the set S of oligonucleotide sequences can avoid mishybridization between them, their complementary sequences, sequences constructed by shifting these sequences, and sequences produced by ligation of the oligonucleotide sequences, of their complementary sequences, and of the oligonucleotide sequences and their complementary sequences ("1"), a set S of oligonucleotide sequences of predetermined length n (n is an integer 3 or more), wherein each of oligonucleotide sequences in the set S induces equal to or more than a fixed, predetermined number of mismatches against any of oligonucleotide sequences in the set S, a reverse sequence of each of oligonucleotide sequences in the set S, sequences constructed by shifting these sequences, and sequences produced by ligation of the oligonucleotide sequences, of their reverse sequences, and of the oligonucleotide sequences and their reverse sequences, and wherein the set S of oligonucleotide sequences can avoid mishybridization between them, their reverse sequences, sequences constructed by shifting these sequences, and sequences produced by ligation of the oligonucleotide sequences, of their reverse sequences, and of the oligonucleotide sequences and their reverse sequences ("2"), the set S of oligonucleotide sequences according to "1" It or "2", which comprises oligonucleotide sequences of predetermined length n (n is an integer 6 or more) ("3") , the set S of oligonucleotide sequences according to any one of "1" to "3", wherein the set S of oligonucleotide sequences of predetermined length n is a set S of oligonucleotide sequences of length 32 or less ("4"), the set S of oligonucleotide sequences according to any one of "1" to "4", wherein the predetermined number of mismatches is equal to or more than one-fourth of the sequence length n ("5"), the set S of oligonucleotide sequences according to any one of "1" to "5", wherein the set S of oligonucleotide sequences is a set of oligonucleotide sequences that contains or never contains a particular subsequence ("6"), and the set S of oligonucleotide sequences according to "6", wherein the particular subsequence is a restriction site ("7").

The present invention also relates to a method for designing the set S of oligonucleotide sequences according to "3", comprising the following steps: 1) Select a binary string (GC template) such that its Hamming distance to its reverse sequence, to its block shift, and its Hamming distance to the overlap part of its tandem concatenation, its concatenation with its reverse sequence, and the tandem concatenation of its reverse sequence, is equal to or above the predetermined value k, and in the following, an oligonucleotide sequence of length n is specified by the binary string of 0 and 1 (GC template) of predetermined length L (L is an integer 6 or more), meaning that the positions of G or C ([GC]), or A or T ([AT]) are fixed; 2) Combine the codewords of any error correcting code with the selected GC template to specify a set of oligonucleotide sequences that induce at least k mismatches between any of them ("8"), a method for designing the set S of oligonucleotide sequences according to "1" or "2", comprising the following steps: 1) Select a binary string (AG template) such that its Hamming distance to its reverse inverted sequence, to its block shift, and its Hamming distance to the overlap part of its tandem concatenation, its concatenation with its reverse inverted sequence, and the tandem concatenation of its reverse inverted sequence, is equal to or above the predetermined value k, and in the following, an oligonucleotide sequence of length n is specified by the binary string of 0 and 1 (AG template) of predetermined length L (L is an integer 6 or more), meaning that the positions of A or G ([AG]), or C or T ([CT]) are fixed; 2) Combine the codewords of any error correcting constant-weight code with the selected AG template to specify a set of oligonucleotide sequences that induce at least k mismatches between any of them ("9"), the method for designing a set S of oligonucleotide sequences according to "8" or "9", wherein any of these oligonucleotide sequences, of which Hamming distance is equal to or above k, induces at least k mismatches against any of the sequences in the set S, their complementary sequences, sequences constructed by shifting these sequences, and the sequences produced by ligation of sequences in the set S, of their complementary sequences, and of the sequences and their complementary sequences, and wherein the sequences in the set S can avoid mishybridization between them, their complementary sequences, or sequences constructed by shifting these sequences, and sequences produced by ligation of sequences in the set S, of their complementary sequences, and of the sequences and their complementary sequences ("10" ) , the method for designing a set S of oligonucleotide sequences according to "8" or "9", wherein any of these oligonucleotide sequences, of which Hamming distance is equal to or above k, induces at least k mismatches against any of the sequences in the set S, their reverse sequences, sequences constructed by shifting these sequences, and the sequences produced by ligation of sequences in the set S, of their reverse sequences, and of the sequences and their reverse sequences, and wherein the sequences in the set S can avoid mishybridization between them, their reverse sequences, or sequences constructed by shifting these sequences, and sequences produced by ligation of sequences in the set S, of their reverse sequences, and of the sequences and their reverse sequences ("11"), the method for designing a set S of oligonucleotide sequences according to any one of "7" to "9", wherein the set S of oligonucleotide sequences of predetermined length n is a set S of oligonucleotide sequences of length 32 or less ("12"), the method for designing a set S of oligonucleotide sequences according to any one of "8" to "12", wherein the predetermined value k is one-fourth of L or more ("13"), the method for designing a set S of oligonucleotide sequences according to any one of "8" to "13", wherein the set S of oligonucleotide sequences is a set of oligonucleotide sequences that contains or never contains a particular subsequence ("14"), the method for designing a set S of oligonucleotide sequences according to "14", wherein the particular subsequence is a restriction site ("15"), and the method for designing a set S of oligonucleotide sequences according to any one of "8" to "15", wherein the codewords of an error correcting code are selected from Hamming codes, BCH codes, maximum-length codes, Golay codes, Reed-Muller codes, Reed-Solomon codes, Hadamard codes, Preparata codes, reversible codes, or constant-weight codes ("16").

The present invention further relates to a method for designing a GC template used for constructing the set S of oligonucleotide sequences according to "3", by selecting a GC template so that its Hamming distance to its reverse sequence, to its block shift, and its Hamming distance to the overlap part of its tandem concatenation, its concatenation with its reverse sequence, and the tandem concatenation of its reverse sequence, is equal to or above the predetermined value k, and wherein an oligonucleotide sequence of length n is specified by the binary string of 0 and 1 (GC template) of predetermined length L (L is an integer 6 or more), meaning that the positions of G or C ([GC]), or A or T ([AT]) are fixed ("17"), the method for designing a GC template according to "17", wherein the GC template of predetermined length L is a GC template of length 32 or less ("18"), the method for designing a GC template according to "17" or "18", wherein the predetermined value k is one-fourth of L or more ("19"), the method for designing a GC template according to "18", wherein the GC template shows 2, 4, 6, 7, 8, 9, 10, 11, or 12, as the predetermined value k, when the length L of the GC template is 6 to 10, 11 to 15, 16 to 18, 19, 20 to 22 and 24, 23 and 25, 26 and 27, 28 and 29, or 30 to 32, respectively ("20"), the method for designing a GC template according to any one of "17" to "20" , wherein the set S of oligonucleotide sequences is a set of oligonucleotide sequences that contains or never contains a particular subsequence ("21") , and the method for designing a GC template according to "21", wherein the particular subsequence is a restriction site ("22").

The present invention also relates to a method for designing an AG template used for constructing the set S of oligonucleotide sequences according to "1" or "2" , by selecting an AG template so that its Hamming distance to its reverse inverted sequence, to its block shift, and its Hamming distance to the overlap part of its tandem concatenation, its concatenation with its reverse inverted sequence, and the tandem concatenation of its reverse inverted sequence, is equal to or above the predetermined value k, and wherein an oligonucleotide sequence of length n is specified by the binary string of 0 and 1 (AG template) of predetermined length L (L is an integer 6 or more), meaning that the positions of A or G ([AG]), or C or T {[CT]) are fixed ("23"), the method for designing an AG template according to "23", wherein the AG template of predetermined length L is an AG template of length 32 or less ("24"), the method for designing an AG template according to "23" or "24", wherein the predetermined value k is one-fourth of L or more ("25") , the method for designing an AG template according to "23", wherein the AG template shows 1, 2, 3, 4, 5, 6, 7, 8, 9, 10, 11, 12, 13, or 13, as the predetermined value k, when the length L of the AG template is 3 to 5, 6 to 8, 9, 10 to 12, 13 and 14, 15 to 18, 19, 20 to 22, 23, 24 to 26, 27, 28 to 30, 31, or 32, respectively ("26"), the method for designing an AG template according to any one of "23" to "26", wherein the set S of oligonucleotide sequences is a set of oligonucleotide sequences that contains or never contains a particular subsequence ("27"), and the method for designing an AG template according to "27", wherein the particular subsequence is a restriction site ("28").

The present invention still further relates to DNA or RNA chips which contain the set S of oligonucleotide sequences according to any one of "1" to "7" ("29"), DNA or RNA tags which contain the set S of oligonucleotide sequences according to any one of "1" to "7" ("30"), DNA or RNA computing systems which use the set S of oligonucleotide sequences according to any one of "1" to "7" ('31'), and DNA or RNA probes selected from the set S of oligonucleotide sequences according to any one of "1" to "7" ("32").

## Brief Description of Drawings

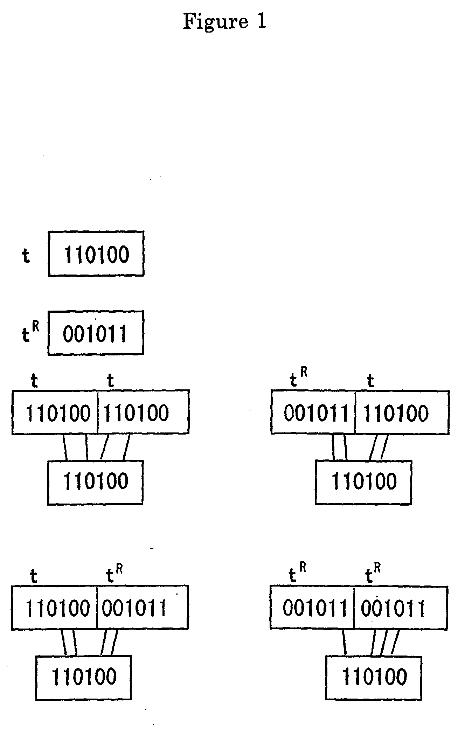

Fig. 1 is a view showing that when GC template t of the present invention, which is 110100, is used, then the Hamming distance minimum value MD (t) equals 2, regardless of the way the GC template t is shifted to ligated sequences.

## Best Mode of Carrying Out the Invention

The set S of oligonucleotide sequences (hereinafter sometimes referred to as "P sequence") of the present invention is not particularly limited as long as it is a set of orthogonal sequences that comprises a set S of P sequences of predetermined length n (in case of GC templates, n is an integer 6 or more, in case of AG templates, n is an integer 3 or more), wherein each of P sequences in the set S induces equal to or more than a fixed, predetermined number of mismatches against any of P sequences in the set S, a complementary sequence (hereinafter sometimes referred to as "$P^C$ sequence") or reverse sequences (hereinafter sometimes referred to as "$P^R$ sequence") of each of P sequences in the set S, sequences constructed by shifting these sequences, and sequences produced by ligation of these P sequences, of $P^C$ sequences or $P^R$ sequences, and of the P sequences and $P^C$ sequences or $P^R$ sequences in the set S. The set S of P sequences can avoid mishybridization between them, $P^C$ sequences or $P^R$ sequences, sequences constructed by shifting these sequences, and sequences produced by ligation of the P sequences, of their complementary sequences, and of the P sequences and $P^C$ sequences or $P^R$ sequences in the set S. The above-mentioned oligonucleotide sequences include DNA sequences and RNA sequences. In addition, though the upper limit of the predetermined length n of the oligonucleotide sequences (in case of GC templates, n is an integer 6 or more, in case of AG templates, n is an integer 3 or more) is not particularly defined, it is normally 100 bases, preferably 32 bases in consideration of the use as a primer in PCR or a DNA tip, on the other hand, when the predetermined length is 5 or less (in case of GC templates), or 2 or less (in case of AG templates), the set S of oligonucleotide sequences of the present invention cannot be obtained. The set S of oligonucleotide sequences, which is a target of the present invention, conveniently includes subsets of the set S. Hereinafter, it is described how the set S inducing mismatches is designed with the use of a GC template mainly, focusing the case where the oligonucleotide sequence is a DNA sequence, including the case of complementary sequences.

The P sequences in the set S of the present invention designed by using a GC template not only induce mismatches of predetermined value or more between the sequences themselves, and between the P sequences and other P sequences in the set S, in both cases where sequences are shifted (sequences are staggered) and not shifted, and can avoid mishybridization, but also induce mismatches of predetermined value or more between the P sequences and $P^C$ sequences which are complementary sequences of each of other oligonucleotide sequences (excluding the P sequences themselves) in the set S, that is, $P^C$ sequences constructed by substituting A, T, G and C in the P sequences with T, A, C and G respectively, and reversing the direction of 5' and 3', in both cases where sequences are shifted and not shifted, and can avoid mishybridization, and induce mismatches of predetermined value or more between the P sequences and oligonucleotide sequences constructed by ligating each of oligonucleotide sequences in the set S, that is, ligated sequences of P sequences, and ligated sequences of $P^C$ sequences, ligated sequences of P sequences and $P^C$ sequences, ligated sequences of $P^C$ sequences and P sequences, etc. , and can avoid mishybridization. Here, mismatch means a pairing with bases other than complementary bases in hybridization, and as mismatches of predetermined value or more, there is no particular limitation as long as it is the number of mismatches with which mishybridization can be avoided, however, it is preferable that mismatches are one-fifth or more, more preferably one-fourth or more, and most preferably one-third or more of predetermined length n (n is an integer 6 or more) of oligonucleotide sequences.

The P sequences in the set S of the present invention not only induce mismatches of predetermined value or more between the sequences themselves, and between the P sequences and other P sequences in the set S, in both cases where sequences are shifted (sequences are staggered) and not shifted and can avoid mishybridization, but also induce mismatches of predetermined value or more between the P sequences and $P^R$ sequences which are reverse sequences of each of P sequences in the set S, that is, sequences (for example, TCAGTTAA) whose 5' side and 3' side are 3' side and 5' side of 5' $\rightarrow$ 3' sequences of (for example, AATTGACT) in the P sequences, respectively, in both cases where sequences are shifted and not shifted, and can avoid mishybridization, and indece mismatches of predetermined value or more between ligated sequences of P sequences, and ligated sequences of $P^R$ sequences, ligated sequences of P sequences and $P^R$ sequences, ligated sequences of $P^R$ sequences and P sequences, etc., and can avoid mishybridization.

Further, it is preferable that the oligonucleotide sequences that compose the set S of the present invention can be operated as oligonucleotide sequences that contain or never contain particular subsequences. Examples of the particular subsequences include restriction sites; expression signal sequences including poly A portions of RNA, ATG which is a translation initiation codon, TAA, TAG, TGA, etc. which are stop codons; consensus sequences GCCAATCT, ATGCAAAT, recognized by transcription factors, and optional DNA sequence signal such as base sequences encoding variable regions of antibodies.

The set S of oligonucleotide sequences of the present invention mentioned above can be usually designed in two steps. The first step is a step of designing a GC template with the use of the Hamming distance, and in the next step, the set S of oligonucleotide sequences of the present invention as an object can be designed from the set of oligonucleotide sequences represented by the designed GC template by using the theory of error correcting codes. Since DNA sequences can be sequences comprising G or C [GC], or A or T [AT], it is determined in the first step whether each of the positions of sequences is [GC] or [AT]. This position is represented by a GC template comprising 0 and 1; $b_1\ b_2 ... b_i\ (b_i \in \{0, 1\})$, and 1 and 0 mean [AT] and [GC], respectively, or , 1 and 0 mean [GC] and [AT], respectively. Therefore, not $4^L$ kinds but $2^L$ kinds of sequences are represented by a GC template of length L. In the next step, base sequences are determined by specifically substituting the position 1 of a GC template with bases at [AT], and the position 0 with bases at [GC], or the position 1 with bases at [GC], and the position 0 with bases at [AT].

The Hamming distance mentioned above is used as a scale for similarity between sequences. For example, the Hamming distance between two strings $x = x_1, x_2, ... x_n$ and $y = y_1, y_2, ... y_n$ is defined as the number of index i that complies with the condition of $x_i \neq y_i$. In addition, as mishybridization between DNA sequences can be occurred even when sequences are shifted (staggered), it is necessary to consider the Hamming distance in the case where sequences are shifted. Since "shift" occurs when one sequence is longer than the other, in case of $|x| < |y|$, the Hamming distance between the two strings is made to be the minimum value of the Hamming distance between x and each of $(|y| - |x| + 1)$ subsequences of length $|x|$ contained in y. The Hamming distance indicated by this minimum value can be represented by H (x, y).

Next, function MD (abbreviation of min distance) against a GC template t is considered in order to obtain the Hamming distance between a GC template t and ligated sequences of the GC templates t, ligated sequences of reverse sequences $t^R$ of the GC templates t, ligated sequences of the GC templates t and reverse sequences $t^R$. The reverse sequence $t^R$ means a sequence wherein a binary string of the GC template t is aligned reversely. As the Hamming distance between a GC template t and a GC template t, its reverse sequence $t^R$, which are sequences at both outer sides of ligated sequences, is already obtained, it is suffice to consider sequences wherein one letter each is deleted from both ends of ligated sequences when obtaining minimum value of the Hamming distance by shifting GC templates t against ligated sequences, consequently, it is convenient to use a symbol [] in a mathematical formula of MD (t). The meaning of symbol [] is: $[S_1\, S_2\, S_3\, ...\, S_{m-1}\, S_m] = S_2\, ...\, S_{m-1}$, that is, it means a sequence wherein one letter each is deleted from both ends. Therefore, the minimum distance MD (t) of the Hamming distance between GC templates t and ligated sequences is represented by the following formula.

$$MD\ (t) = \min \{H\ (t,\ t^R),\ H\ (t,\ [tt]),\ H\ (t,\ [tt^R]),\ H\ (t,\ [t^Rt]\ ,\ H\ (t,\ [t^Rt^R])\}.$$

Consequently, in case where MD (t) = k (k $\geqq$ 0) for a GC template t, at least Hamming distance k is ensured for sequences [tt], [tt$^R$], [t$^R$t], [t$^R$t$^R$], including ligating parts thereof, wherein one letter each is deleted from both ends of ligated sequences, when a GC template t is shifted against ligated sequences. Fig. 1 shows that when GC template t = 110100, then MD (t) = 2. In this case, reverse sequence t$^R$ = 001011, [tt] = 1010011010, [tt$^R$] = 1010000101, [t$^R$t] = 0101111010, [t$^R$t$^R$] = 0101100101, and Fig. 1 shows the case where each Hamming distance is 2. As seen from Fig. 1, GC template t = 110100 cannot shorten the Hamming distance beyond 2 regardless of the way of shifting, therefore, it would be defined that MD (t) = 2.

Thus, the method for designing a GC template of the present invention is used at the first step of constructing the set S of oligonucleotide sequences of the present invention. As seen from the above explanation, the method for designing a GC template of the present invention is not particularly limited as long as it is a method comprising selection of GC templates such that its Hamming distance to its reverse sequence, to its block shift, and its Hamming distance to the overlap part of its tandem concatenation, its concatenation with its reverse sequence, and the tandem concatenation of its reverse sequence, is equal to or above the predetermined value k. In the following, an oligonucleotide sequence of length n is specified by the binary string of 0 and 1 (GC template) of predetermined length L (L is an integer 6 or more), meaning that the positions of G or C ([GC]), or A or T ([AT]) are fixed. However, the length L of GC template is 6 or more, preferably 6 to 100, more preferably 6 to 32, most preferably around 20, which is often used in experiments of molecular biology. If the length is 5 or less, the one having desired Hamming distance cannot be obtained. By using the GC template having the length L, a set S of oligonucleotide sequences of corresponding length n can be obtained. Further, the predetermined value k is not particularly limited as long as it is a value that allows oligonucleotide sequences constructed from the GC template to be the oligonucleotide sequences of the present invention that can avoid mishybridization. The value is preferably one-fifth or more, more preferably one-fourth or more, most preferably one-third or more of the length L of the GC template.

In general, when the length L is increased or MD value (k value) is decreased, many more GC templates will exist, however, a GC template of predetermined length and having the greatest k value (MD value) is particularly important. Examples of GC templates of length L = 6 to 32 and having the greatest k value (MD value) include; GC templates having length L = 6 to 10, 11 to 15, 16 to 18, 19, 20 to 22 and 24, 23 and 25, 26 and 27, 28 and 29, 30 to 32, and the predetermined value k = 2, 4, 6, 7, 8, 9, 10, 11, 12, respectively. The maximum value of the predetermined value k in the GC templates of length L = 6 to 32, the number of GC templates having the maximum value, and specific examples are shown in [Table 1]. In addition, the shortest GC templates that fulfill specific MD value (k value) are shown in [Table 2]. Further, specific examples for GC templates of length L = 11 to 27 and those for GC templates of length L = 28 to 30 are shown in [Table 3] and [Table 4], respectively. In [Table 2], GC templates are enumerated excluding the ones that have the same reverse sequences or sequences wherein 0 and 1 are reversed, and in [Table 3] and [Table 4], "items" are the numbers after omitting GC templates that become identical by cyclic shift.

(Table 1)

| Length L | Distance k | The number of templates | Specific examples |
|---|---|---|---|
| 6 | 2 | 1 | 110100 |
| 7 | 2 | 6 | 0101100 |
| 8 | 2 | 18 | 11001000 |
| 9 | 2 | 45 | 111010000 |
| 10 | 2 | 148 | 0111101000 |
| 11 | 4 | 3 | 11000100101 |
| 12 | 4 | 31 | 111000100101 |
| 13 | 4 | 109 | 0101100010000 |
| 14 | 4 | 496 | 10011010100000 |
| 15 | 4 | 1426 | 111000001010011 |
| 16 | 6 | 12 | 1110001101000100 |
| 17 | 6 | 67 | 00101110010110000 |
| 18 | 6 | 1043 | 111001000110000111 |
| 19 | 7 | 5 | 1111001001100001010 |
| 20 | 8 | 6 | 11101110001000110100 |
| 21 | 8 | 19 | 111101010000001101100 |
| 22 | 8 | 982 | 1110001011101101000000 |
| 23 | 9 | 3 | 11100000101001100110101 |
| 24 | 8 | 71007 | 111101001011000000001111 |
| 25 | 9 | 88 | 1010111100000001010011001 |
| 26 | 10 | 731 | 10101110110110001111000000 |
| 27 | 10 | 4980 | 111010011010100100000010111 |
| 28 | 11 | 18 | 1111101011000000111001010001 |
| 29 | 11 | 1 | 11101110110100100010001110000 |
| 30 | 12 | 178 | 001011111000101101011001100000 |
| 31 | 12 | 2615 | 1001110110111100010101110000000 |
| 32 | 12 | 191945 | 11010011110101000110111000000000 |

(Table 2)

| MD value | Length | Templates | | |
|---|---|---|---|---|
| 2 | 6 | 110100 | | |
| 4 | 11 | 01000111010, | 00111011010, | 01110100100 |
| 6 | 16 | 1011001000010101 | 1011100000100101 | 1011100010000101 |
| | | 1001111000001001 | 0101101110000010 | 0111101000001100 |
| | | 1110001101000100 | 0011010011101000 | 1011000111001000 |
| | | 0101101110001000 | 0101111000110000 | 1100101101010000 |
| 7 | 19 | 0111101010000110110, | 1001100001010111100, | |
| | | 1010111100110110000, | 1010111100100110000, | |
| | | 1101100111101010000 | | |
| 8 | 20 | 1101001110111000100 0, | 0111101001100110100 0, | |
| | | 1101110100010011100 0, | 1110001101110100010 0, | |
| | | 1110111000100011010 0, | 1110100110011010000 1 | |

(Table 3)

| Length (d) | |
|---|---|
| 11 (4) | 01110100100 |
| 12 (4) | 000111011010 |
| | 001011100110 |
| | 001111010100 |
| | 010011011100 |
| | 010111100010 |
| | 011010100110 |
| | 101001100000 |
| | 101100001000 |
| | 111001011000 |
| 13 (4) | 0000101100010 |
| | 0000111011010 |
| 22 | 0001011001110 |
| items | 0001011100110 |
| | 0001110110010 |
| | 0010010011100 |
| | 0010100101110 |
| | 0010100111010 |
| | 0010110010110 |
| | 0011110101000 |
| | 0100010111000 |
| | 0110010100000 |
| | 0110011110000 |
| | 0110101001100 |
| | 1000110110100 |
| | 1000111010000 |
| | 1001011100000 |
| | 1010010011000 |
| | 1010110010000 |
| | 1010110110000 |
| | 1010111001000 |
| | 1101100101000 |
| 14 (4) | 79 items |
| 15 (4) | 180 items |
| 16 (6) | 0001100011110100 |
| | 0010011100011010 |
| | 0011010011101000 |
| | 0101000010011011 |
| | 0101101110001000 |
| | 1000001110110100 |
| | 1001111000001001 |
| | 1100101101010000 |
| 17 (6) | 00001000100110111 |
| | 00001011100101100 |
| 26 | 00010010101100110 |
| items | 00010101011011000 |
| | 00011000111110100 |
| | 00011101101001000 |
| | 00100101011111000 |
| | 00100111000101100 |
| | 01000011110110010 |
| | 01000110011110000 |
| | 01001011000101110 |
| | 01001011101100010 |
| | 01001111010101000 |
| | 01010000010011011 |
| | 01100011110100000 |
| | 01110001001101010 |
| | 01110101100101000 |
| | 10000011101010010 |
| | 10011000010111100 |
| | 10110001110010000 |
| | 10110010111000100 |
| | 10111001100010100 |
| | 11000111011010000 |
| | 11010100110100000 |
| | 11101010001100100 |
| | 11110010001100001 |
| 18 (6) | 209 items |
| 19 (7) | 1010111100100110000 |
| 20 (8) | 10000101100110010111 |
| | 11010011101110001000 |
| | 11011101000100111000 |
| 21 (8) | 000101101001111001100 |
| | 001001011011100010110 |
| | 010101000001110011011 |
| | 010101111000110110110 |
| | 011010001010011101100 |
| | 011110100000100110110 |
| | 100110110101110000010 |
| | 101000001100010011110 |
| | 101011110011011000000 |
| | 111100110000011010100 |
| 22 (8) | 409 items |
| 23 (9) | 0111101011001100101 0000 |
| 24 (8) | 10760 items |
| 25 (9) | 0000100011011010011101010 |
| | 0000101011000110110100110 |
| 20 | 0000110010101100011110010 |
| items | 0001000101101001011100110 |
| | 0001100111100101011010000 |
| | 0010000110110001111010100 |
| | 0010011100001101101010100 |
| | 0010100110001101011110000 |
| | 0011110101100110010100000 |
| | 0101000001100110001111010 |
| | 0101001101001110110001000 |
| | 0101110011010010100110000 |
| | 0110011100010100001011010 |
| | 0110100011000110100000111 |
| | 0111100110010000110101000 |
| | 1000010100011001110110 |
| | 1011001110010101011000000 |
| | 1101010011100110100010000 |
| | 1110010100110011010100000 |
| | 1110011001000001010110100 |
| 26 (10) | 330 items |
| 27 (10) | 2272 items |

(Table 4)

28
(11)

29 (11)
30 (12)

157
items

(Table 4) contd.

The GC template sequences enumerated in [Table 1] to [Table 4], etc., can be selected by searching exhaustively all patterns from sequences comprising only 0 to sequences comprising only 1, by a person skilled in the art.

However, there is no need to search all $2^L$ patterns to find a GC template of length L. It is suffice to take into account the GC templates wherein bit 1 contained therein is L/2 or less because GC templates whose bits 01 are reversed have same property. In addition, from the constraint of the number of mismatches, it is shown that in case where the minimum distance is d, the number of bit 1 is at least (L-sqrt $(L^2-2dL)$)/2 (sqrt means square root). The GC templates can be efficiently obtained by using these constraints additionally. Further, when GC templates are designed such that the set S of oligonucleotide sequences constructed from GC templates is made to be a set of oligonucleotide sequences that contains or never contains particular subsequences such as restriction sites mentioned above, such designing corresponds to the narrowing of the space for exhaustive search, and therefore it contributes easier designing.

The set S of oligonucleotide sequences of the present invention can be designed by the step following the design of GC templates with the use of the Hamming distance mentioned above, which is the step using the theory of error correcting codes, that is, by combining codewords of any error correcting code with the designed GC templates to specify a set of oligonucleotide sequences, and by specifically substituting the positions 1 and 0 of GC template with bases of [AT] and [GC], or the positions 1 and 0 of GC template with bases of [GC] and [AT], respectively. As the codewords of error correcting codes mentioned above, any codewords can be used as long as they are known codewords of error correcting codes, and specific examples include Hamming codes, BCH codes, maximum-length codes, Golay codes, Reed-Muller codes, Reed-Solomon codes, Hadamard codes, Preparata codes, and reversible codes.

The motive for using the theory of error correcting codes is to ensure mismatches to complementary sequences in case where there occurs no shift (see claim 1). Therefore, as to the set S inducing mismatches in consideration of reverse sequence as well (see claim 2), it is not always necessary to use error correcting codes. Error correcting codes are a set of codewords wherein there are at least a certain number of mismatches between optional codewords. In case of preventing mishybridization between a set S and a set of reverse sequences thereof, it is only necessary to apply a set of codewords wherein there are at least a certain number of matches (not mismatches) between optional codewords. As for the set S of oligonucleotide sequences of the present invention, information of the codewords and GC templates are reflected on the sequences. Therefore, it is suffice to use error correcting codes maintaining the Hamming distance (the number of mismatches) k or more in order to ensure k mismatches to complementary sequences, and it is suffice to use codes maintaining the number of matches k or more in order to ensure k mismatches to reverse sequences.

In the theory of error correcting codes, codes wherein a redundant bit for detecting and correcting errors, which is called parity bit, is added to a given information bit to make the Hamming distance between optional codewords above a certain value, have been developed. The minimum value of the Hamming distance between codewords is called minimum distance. As the object of the code theory is to design the one that maintains the minimum distance largely and contains many codewords, there are many codes that meet the purpose of the present invention. For example, there are 4096 words of Golay code of code length 23 and minimum distance 7. With the use of this code, it is possible to design 4096 oligonucloetides for one GC template of length 23 (MD value is up to 9).

Next, it is explained with specific example of the combination of error correcting codes and GC templates. As for GC templates, the Hamming code of minimum distance 3 and length L = 7 is applied to 1101000 (upper) of MD (t) = 2 and length L = 7. It is ensured that the sequences thus constructed have at least two mismatches (in case shift does not occur, three mismatches) to any ligation or shift, on each side. For instance, if it is defined that 00 is A, 01 is T, 10 is G, and 11 is C, a set of 16 DNA sequences comprising 7 bases shown in [Table 5] whose GC content is 3/7 is given. Further, if it is defined that 00 is G, 01 is C, 10 is A, and 11 is T, a set of 16 DNA sequences comprising 7 bases shown in [Table 6] whose GC content is 4/7 is given.

(Table 5)

| 1101000<br>0000000<br>GGAGAAA | 1101000<br>1000101<br>CGAGTAT | 1101000<br>0100111<br>GCAGTTT | 1101000<br>1100010<br>CCAGATA |
|---|---|---|---|
| 1101000<br>0010110<br>GGTGTTA | 1101000<br>1010011<br>CGTGATT | 1101000<br>0110001<br>GCTGAAT | 1101000<br>1110100<br>CCTGTAA |
| 1101000<br>0001011<br>GGACATT | 1101000<br>1001110<br>CGACTTA | 1101000<br>0101100<br>GCACTAA | 1101000<br>1101001<br>CCACAAT |

**(Table 6)**

| 1101000<br>0000000<br>AAGAGGG | 1101000<br>1000101<br>TAGACGC | 1101000<br>0100111<br>ATGACCC | 1101000<br>1100010<br>TTGAGCG |
|---|---|---|---|
| 1101000<br>0010110<br>AACACCG | 1101000<br>1010011<br>TACAGCC | 1101000<br>0110001<br>ATCAGGC | 1101000<br>1110100<br>TTCACGG |
| 1101000<br>0001011<br>AAGTGCC | 1101000<br>1001110<br>TAGTCCG | 1101000<br>0101100<br>ATGTCGG | 1101000<br>1101001<br>TTGTGGC |

The method for designing the set S of oligonucleotide sequences of the present invention using GC templates is specifically shown above. The method for designing the set S of oligonucleotide sequences of the present invention is not particularly limited, as seen from the above explanation, as long as it is a method for designing the set S of oligonucleotide sequences comprising the steps of selecting GC templates such that its Hamming distance to its reverse sequence, to its block shift, and its Hamming distance to the overlap part of its tandem concatenation, its concatenation with its reverse sequence, and the tandem concatenation of its reverse sequence, is equal to or above the predetermined value k, and in the following, an oligonucleotide sequence of length n is specified by the binary string of 0 and 1 (GC template) of predetermined length L (L is an integer 6 or more), meaning that the positions of G or C ([GC]), or A or T ([AT]) are fixed; and of combining the codewords of any error correcting code of minimum distance k with the selected GC template to specify a set of oligonucleotide sequences that induce at least k mismatches between any of them. However, the design method wherein the set of oligonucleotide sequences that maintains the Hamming distance k induces equal to or more than a fixed, predetermined number of mismatches against any of P sequences in the set S, a complementary sequence or reverse sequences of each of P sequences in the set S, sequences constructed by shifting these sequences, and sequences produced by ligation of these P sequences, of $P^C$ sequences or $P^R$ sequences, and of the P sequences and $P^C$ sequences or $P^R$ sequences in the set S, and wherein the set S of P sequences can avoid mishybridization between them, $P^C$ sequences or $P^R$ sequences, sequences constructed by shifting these sequences, and sequences produced by ligation of the P sequences, of their complementary sequences, and of the P sequences and $P^C$ sequences or $P^R$ sequences in the set S, is preferable.

Further, in the method for designing a GC template of the present invention, length n of oligonucleotide sequences in the predetermined set S, length L of GC templates, and the predetermined value k are as explained above, and the set S of oligonucleotide sequences is a set of oligonucleotide sequences that contains or never contains particular subsequences as explained above, and Hamming codes, BCH codes, maximum-length codes, Golay codes, Reed-Muller codes etc. can be used as the above-mentioned codewords of any error correcting codes as mentioned above.

So far, GC templates whose binary strings designate [GC], [AT] have been described. As application thereof, a design method using an AG template wherein each position designates A or G ([AG]), or T or C (ETC]) is exemplified. In order to do this, the definition of function MD in the GC template is redefined as follows.

$$MD (t) = min \{H (t,T^R) , H (t, [tt]), H (t, [T^R T^R]), H (t, [tT^R]),$$

$$H ([T^R t])\}$$

Here, symbol T means a binary string constructed by reversing 0 and 1 of all bits of template t (for example, when t = 010101, then T = 101010). The largest difference from GC template resides in the point that when a binary string that maximize this MD value is selected from among binary strings of given length L and this binary string is set to be t, the binary string of t designates [AG] or [TC], therefore, GC content of designed DNA sequences cannot be standardized in case where the binary strings of t are combined with error correcting codewords. In GC templates, position of GC is designated by 01 of the templates and position of AG is designated by 01 of the error correcting codewords. In AG templates, the designation of the positions is reversed. Therefore, it is impossible to standardize GC content with the use of optional error correcting codewords, it is necessary to use error correcting codes called constant-weight codes that have constant number of 1 in codewords. It is more difficult to design the constant-weight

**EP 1 452 598 A1**

codes than generally used codes such as BCH codes or Hadamard codes that can use templates designating [GC] or [AT], but the constant-weight codes can be systematically designed with the use of the result described in reference BSS90 (IEEE Trans. On Information Theory, 36, pp. 1334-1380, 1990).

However, while constraints are imposed on available error correcting codes, it is possible to make the MD value of the templates, that is, the Hamming distance in consideration of shift and ligation, larger than that of the templates designating [GC] or [AT]. Further, it is found that the number of templates that have same MD value will be more than that of the templates designating [GC] or [AT]. The length L of AG template is 3 or more, preferably 3 to 100, more preferably 3 to 32, most preferably around 20, which is often used in experiments of molecular biology. If the length is 2 or less, the one having desired Hamming distance cannot be obtained. Further, the predetermined value k is not particularly limited as long as it is a value that allows oligonucleotide sequences constructed from the AG template to be the oligonucleotide sequences of the present invention that can avoid mishybridization. The value is preferably one-fifth or more, more preferably one-fourth or more, and most preferably one-third or more of the length L of the GC template.

As in the case of GC templates, when the length L is increased or MD value (k value) is decreased, many more AG templates will exist, however, an AG template of predetermined length and having the greatest k value (MD value) is particularly important. Examples of AG template of length L = 3 to 32 and having the greatest k value (MD value) include; AG templates having length L = 3 to 5, 6 to 8, 9, 10 to 12, 13 and 14, 15 to 18, 19, 20 to 22, 23, 24 to 26, 27, 28 to 30, 31, 32 and the predetermined value k = 1, 2, 3, 4, 5, 6, 7, 8, 9, 10, 11, 12, 13, 13, respectively. The maximum value of the predetermined value k in the AG template of length L = 3 to 30, the number of AG templates having the maximum value, and specific examples are shown in [Table 7]. The number of AG templates in [Table 7] contains all templates without omitting templates that become identical by cyclic shift or reversal.

(Table 7)

| Length L | Distance k | The number of templates | Specific examples |
|---|---|---|---|
| 3 | 1 | 4 | 110 |
| 4 | 1 | 8 | 1110 |
| 5 | 1 | 24 | 11110 |
| 6 | 2 | 14 | 111100 |
| 7 | 2 | 32 | 1111100 |
| 8 | 2 | 92 | 11111100 |
| 9 | 3 | 44 | 111110010 |
| 10 | 4 | 4 | 1001111110 |
| 11 | 4 | 20 | 11110011101 |
| 12 | 4 | 358 | 111111011000 |
| 13 | 5 | 20 | 1111110010100 |
| 14 | 5 | 8 | 11111001101011 |
| 15 | 6 | 4 | 111100110111101 |
| 16 | 6 | 232 | 1111111011000110 |
| 17 | 6 | 956 | 11111111011000110 |
| 18 | 6 | 11564 | 111111111100101000 |
| 19 | 7 | 252 | 1111111101001011100 |
| 20 | 8 | 200 | 11111110001110110110 |
| 21 | 8 | 408 | 111111111000101101000 |
| 22 | 8 | 23510 | 1111111111001001110010 |
| 23 | 9 | 848 | 11111111000101100011010 |
| 24 | 10 | 24 | 111111011111000110101100 |
| 25 | 10 | 208 | 1111111110010111010011100 |
| 26 | 10 | 27836 | 11111111111010001110010100 |
| 27 | 11 | 180 | 111101101111100110010101000 |
| 28 | 12 | 12 | 1110101110011001011111101001 |
| 29 | 12 | 52 | 11111110100011100011011101101 |
| 30 | 12 | 23056 | 111111111110001110101101001100 |
| 31 | 13 | 24 | 1000101000001001010011101100000 |
| 32 | 13 | 528 | 10010101100100110001110000000000 |

The case using AG templates and the case using GC templates have a lot in common, for example, in both cases, it is preferable that the set S of oligonucleotide sequences is a set of oligonucleotide sequences that contains or never contains particular subsequences such as restriction sites. Though templates designating [AG] or [TC) have an advantage that they can maintain larger Hamming distance than templates designating [GC] or [AT], the number of the codewords of constant-weight codes is not so many in general. Therefore, from the viewpoint of the number of words that can be designed, GC templates are more flexible and have wide application. Further, GC templates have a great advantage that the melting temperature calculated by the nearest neighbor method used in biological experiments can be standardized because not only GC content but also alignment of GC bases can be standardized in all sequences. Therefore, AG templates can be handled also as one of possible variations.

The set S of oligonucleotide sequences of the present invention can be advantageously used as DNA or RNA tips, or DNA or RNA tags because orthogonalization between sequences makes it difficult to mishybridize with each other even if more than one kinds of oligonucleotide chains are fixed on a substrate in the high density. In addition, the set S of oligonucleotide sequences of the present invention is useful as primers for PCR, etc. because it is difficult to mishybridize with complementary sequences, as well. Further, the set S of oligonucleotide sequences of the present invention can be advantageously used for DNA computing system that comprises the steps of; artificially synthesizing DNA sequences in which various symbol manipulation operating systems such as logical expression and graph structure are recorded, and cutting and pasting the sequences according to protocols of molecular biological experiments, and sequences obtained at the end of the experiments are "calculation results" of the DNA computing, because it has a specific sequence portion such as restriction sites in addition that it is difficult to mishybridize with each other.

**Industrial Applicability**

The method for designing the set S of oligonucleotide sequences of the present invention makes it possible to efficiently and systematically design DNA sequences wherein it is difficult to mishybridize with each other due to the orthogonality of the sequences. Therefore, in biotechnology in general wherein information is written in DNA, the design method of the present invention is an essential technique for reducing experimental errors due to mishybridization of DNA. In addition, sequences that ensure the value of mishybridization can be systematically constructed by combining a set of GC templates obtained by the method for designing a GC template of the present invention and codewords of optional error correcting codes. Further, as the method for designing the set S of oligonucleotide sequences of the present invention fixes the site where GC or AT bases appear, there are following advantages.

(1) As GC content of the sequences can be standardized, physical property (in particular, melting temperature) of the sequences can be easily adjusted.
(2) By searching GC templates that match the sequence pattern, particular subsequences such as restriction sites can be introduced beforehand (optional subsequences can be incorporated into a designated sequence portion by making the portion correspondent to the information bit of error correcting codes).
(3) More than one GC template can be combined and used unless MD value does not decrease even if GC templates are ligated each other.

**Claims**

1. A set S of oligonucleotide sequences of predetermined length n (n is an integer 3 or more), wherein each of oligonucleotide sequences in the set S induces equal to or more than a fixed, predetermined number of mismatches against any of oligonucleotide sequences in the set S, a complementary sequence of each of oligonucleotide sequences in the set S, sequences constructed by shifting these sequences, and sequences produced by ligation of these oligonucleotide sequences, of their complementary sequences, and of the oligonucleotide sequences and their complementary sequences, and wherein the set S of oligonucleotide sequences can avoid mishybridization between them, their complementary sequences, sequences constructed by shifting these sequences, and sequences produced by ligation of the oligonucleotide sequences, of their complementary sequences, and of the oligonucleotide sequences and their complementary sequences.

2. A set S of oligonucleotide sequences of predetermined length n (n is an integer 3 or more), wherein each of oligonucleotide sequences in the set S induces equal to or more than a fixed, predetermined number of mismatches against any of oligonucleotide sequences in the set S, a reverse sequence of each of oligonucleotide sequences in the set S, sequences constructed by shifting these sequences, and sequences produced by ligation of the oligonucleotide sequences, of their reverse sequences, and of the oligonucleotide sequences and their reverse sequences, and wherein the set S of oligonucleotide sequences can avoid mishybridization between them, their reverse sequences, sequences constructed by shifting these sequences, and sequences produced by ligation of the oligonucleotide sequences, of their reverse sequences, and of the oligonucleotide sequences and their reverse sequences.

3. The set S of oligonucleotide sequences according to claim 1 or 2, which comprises oligonucleotide sequences of predetermined length n (n is an integer 6 or more).

4. The set S of oligonucleotide sequences according to any one of claims 1 to 3, wherein the set S of oligonucleotide sequences of predetermined length n is a set S of oligonucleotide sequences of length 32 or less.

5. The set S of oligonucleotide sequences according to any one of claims 1 to 4, wherein the predetermined number of mismatches is equal to or more than one-fourth of the sequence length n.

**6.** The set S of oligonucleotide sequences according to any one of claims 1 to 5, wherein the set S of oligonucleotide sequences is a set of oligonucleotide sequences that contains or never contains a particular subsequence.

**7.** The set S of oligonucleotide sequences according to claim 6, wherein the particular subsequence is a restriction site.

**8.** A method for designing the set S of oligonucleotide sequences according to claim 3, comprising the following steps: 1) Select a binary string (GC template) such that its Hamming distance to its reverse sequence, to its block shift, and its Hamming distance to the overlap part of its tandem concatenation, its concatenation with its reverse sequence, and the tandem concatenation of its reverse sequence, is equal to or above the predetermined value k, and in the following, an oligonucleotide sequence of length n is specified by the binary string of 0 and 1 (GC template) of predetermined length L (L is an integer 6 or more), meaning that the positions of G or C ([GC]), or A or T ([AT]) are fixed; 2) Combine the codewords of any error correcting code with the selected GC template to specify a set of oligonucleotide sequences that induce at least k mismatches between any of them.

**9.** A method for designing the set S of oligonucleotide sequences according to claim 1 or 2, comprising the following steps: 1) Select a binary string (AG template) such that its Hamming distance to its reverse inverted sequence, to its block shift, and its Hamming distance to the overlap part of its tandem concatenation, its concatenation with its reverse inverted sequence, and the tandem concatenation of its reverse inverted sequence, is equal to or above the predetermined value k, and in the following, an oligonucleotide sequence of length n is specified by the binary string of 0 and 1 (AG template) of predetermined length L (L is an integer 6 or more), meaning that the positions of A or G ([AG]), or C or T ([CT]) are fixed; 2) Combine the codewords of any error correcting constant-weight code with the selected AG template to specify a set of oligonucleotide sequences that induce at least k mismatches between any of them.

**10.** The method for designing a set S of oligonucleotide sequences according to claim 8 or 9, wherein any of these oligonucleotide sequences, of which Hamming distance is equal to or above k, induces at least k mismatches against any of the sequences in the set S, their complementary sequences, sequences constructed by shifting these sequences, and the sequences produced by ligation of sequences in the set S, of their complementary sequences, and of the sequences and their complementary sequences, and wherein the sequences in the set S can avoid mishybridization between them, their complementary sequences, or sequences constructed by shifting these sequences, and sequences produced by ligation of sequences in the set S, of their complementary sequences, and of the sequences and their complementary sequences.

**11.** The method for designing a set S of oligonucleotide sequences according to claim 8 or 9, wherein any of these oligonucleotide sequences, of which Hamming distance is equal to or above k, induces at least k mismatches against any of the sequences in the set S, their reverse sequences, sequences constructed by shifting these sequences, and the sequences produced by ligation of sequences in the set S, of their reverse sequences, and of the sequences and their reverse sequences, and wherein the sequences in the set S can avoid mishybridization between them, their reverse sequences, or sequences constructed by shifting these sequences, and sequences produced by ligation of sequences in the set S, of their reverse sequences, and of the sequences and their reverse sequences.

**12.** The method for designing a set S of oligonucleotide sequences according to any one of claims 7 to 9, wherein the set S of oligonucleotide sequences of predetermined length n is a set S of oligonucleotide sequences of length 32 or less.

**13.** The method for designing a set S of oligonucleotide sequences according to any one of claims 8 to 12, wherein the predetermined value k is one-fourth of L or more.

**14.** The method for designing a set S of oligonucleotide sequences according to any one of claims 8 to 13, wherein the set S of oligonucleotide sequences is a set of oligonucleotide sequences that contains or never contains a particular subsequence.

**15.** The method for designing a set S of oligonucleotide sequences according to claim 14, wherein the particular subsequence is a restriction site.

**16.** The method for designing a set S of oligonucleotide sequences according to any one of claims 8 to 15, wherein the codewords of an error correcting code are selected from Hamming codes, BCH codes, maximum-length codes, Golay codes, Reed-Muller codes, Reed-Solomon codes, Hadamard codes, Preparata codes, reversible codes, or constant-weight codes.

**17.** A method for designing a GC template used for constructing the set S of oligonucleotide sequences according to claim 3, by selecting a GC template so that its Hamming distance to its reverse sequence, to its block shift, and its Hamming distance to the overlap part of its tandem concatenation, its concatenation with its reverse sequence, and the tandem concatenation of its reverse sequence, is equal to or above the predetermined value k, and wherein an oligonucleotide sequence of length n is specified by the binary string of 0 and 1 (GC template) of predetermined length L (L is an integer 6 or more), meaning that the positions of G or C ([GC]), or A or T ([AT]) are fixed.

**18.** The method for designing a GC template according to claim 17, wherein the GC template of predetermined length L is a GC template of length 32 or less.

**19.** The method for designing a GC template according to claim 17 or 18, wherein the predetermined value k is one-fourth of L or more.

**20.** The method for designing a GC template according to claim 18, wherein the GC template shows 2, 4, 6, 7, 8, 9, 10, 11, or 12, as the predetermined value k, when the length L of the GC template is 6 to 10, 11 to 15, 16 to 18, 19, 20 to 22 and 24, 23 and 25, 26 and 27, 28 and 29, or 30 to 32, respectively.

**21.** The method for designing a GC template according to any one of claims 17 to 20, wherein the set S of oligonucleotide sequences is a set of oligonucleotide sequences that contains or never contains a particular subsequence.

**22.** The method for designing a GC template according to claim 21, wherein the particular subsequence is a restriction site.

**23.** A method for designing an AG template used for constructing the set S of oligonucleotide sequences according to claim 1 or 2, by selecting an AG template so that its Hamming distance to its reverse inverted sequence, to its block shift, and its Hamming distance to the overlap part of its tandem concatenation, its concatenation with its reverse inverted sequence, and the tandem concatenation of its reverse inverted sequence, is equal to or above the predetermined value k, and wherein an oligonucleotide sequence of length n is specified by the binary string of 0 and 1 (AG template) of predetermined length L (L is an integer 6 or more), meaning that the positions of A or G ([AG]), or C or T ([CT]) are fixed.

**24.** The method for designing an AG template according to claim 23, wherein the AG template of predetermined length L is an AG template of length 32 or less.

**25.** The method for designing an AG template according to claim 23 or 24, wherein the predetermined value k is one-fourth of L or more.

**26.** The method for designing an AG template according to claim 23, wherein the AG template shows 1, 2, 3, 4, 5, 6, 7, 8, 9, 10, 11, 12, 13, or 13, as the predetermined value k, when the length L of the AG template is 3 to 5, 6 to 8, 9, 10 to 12, 13 and 14, 15 to 18, 19, 20 to 22, 23, 24 to 26, 27, 28 to 30, 31, or 32, respectively.

**27.** The method for designing an AG template according to any one of claims 23 to 26, wherein the set S of oligonucleotide sequences is a set of oligonucleotide sequences that contains or never contains a particular subsequence.

**28.** The method for designing an AG template according to claim 27, wherein the particular subsequence is a restriction site.

**29.** DNA or RNA chips which contain the set S of oligonucleotide sequences according to any one of claims 1 to 7.

**30.** DNA or RNA tags which contain the set S of oligonucleotide sequences according to any one of claims 1 to 7.

**31.** DNA or RNA computing systems which use the set S of oligonucleotide sequences according to any one of claims 1 to 7.

**32.** DNA or RNA probes selected from the set S of oligonucleotide sequences according to any one of claims 1 to 7.

# Figure 1

t | 110100 |

$t^R$ | 001011 |

t      t
| 110100 | 110100 |
| 110100 |

$t^R$     t
| 001011 | 110100 |
| 110100 |

t      $t^R$
| 110100 | 001011 |
| 110100 |

$t^R$     $t^R$
| 001011 | 001011 |
| 110100 |

**EP 1 452 598 A1**

## INTERNATIONAL SEARCH REPORT

| | International application No. |
|---|---|
| | PCT/JP02/11163 |

**A. CLASSIFICATION OF SUBJECT MATTER**
Int.Cl⁷ C12N15/10, C12Q1/68, G01N33/53, G01N33/566, G06F17/50

According to International Patent Classification (IPC) or to both national classification and IPC

**B. FIELDS SEARCHED**

Minimum documentation searched (classification system followed by classification symbols)
Int.Cl⁷ C12N15/10, C12Q1/68, G01N33/53, G01N33/566, G06F17/50

Documentation searched other than minimum documentation to the extent that such documents are included in the fields searched

Electronic data base consulted during the international search (name of data base and, where practicable, search terms used)
WPI(DIALOG), BIOSIS(DIALOG), JICST FILE(JOIS)

**C. DOCUMENTS CONSIDERED TO BE RELEVANT**

| Category* | Citation of document, with indication, where appropriate, of the relevant passages | Relevant to claim No. |
|---|---|---|
| X<br>A | WO 96/12014 A1 (LYNX THERAPEUTICS INC.),<br>25 April, 1996 (25.04.96),<br>& AU 9642778 A        & US 5604097 A<br>& US 5635400 A        & NO 9701644 A<br>& FI 9701473 A        & US 5654413 A<br>& EP 793718 A1        & CZ 9700866 A3<br>& JP 10-507357 A      & KR 97707279 A<br>& HU 77916 T          & AU 9952663 A | 1-7,29-32<br>8-28 |
| X<br>A | WO 96/41011 A1 (LYNX THERAPEUTICS INC.),<br>19 December, 1996 (19.12.96),<br>& AU 9661020 A        & NO 9705744 A<br>& EP 832287 A1        & CZ 9703926 A3<br>& CN 1193357 A        & HU 9900910 A2<br>& JP 11-507528 A      & KR 99022543 A | 1-7,29-32<br>8-28 |

☒ Further documents are listed in the continuation of Box C. ☐ See patent family annex.

| * | Special categories of cited documents: |
|---|---|
| "A" | document defining the general state of the art which is not considered to be of particular relevance |
| "E" | earlier document but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 06 February, 2003 (06.02.03) | 25 February, 2003 (25.02.03) |

| Name and mailing address of the ISA/ | Authorized officer |
|---|---|
| Japanese Patent Office | |
| Facsimile No. | Telephone No. |

Form PCT/ISA/210 (second sheet) (July 1998)

22

**EP 1 452 598 A1**

| INTERNATIONAL SEARCH REPORT | International application No. |
|---|---|
| | PCT/JP02/11163 |

C (Continuation). DOCUMENTS CONSIDERED TO BE RELEVANT

| Category* | Citation of document, with indication, where appropriate, of the relevant passages | Relevant to claim No. |
|---|---|---|
| X<br>A | WO 94/11837 A1 (Hitachi Chem. Co., Ltd.),<br>26 May, 1994 (26.05.94),<br>& EP 677194 A1      & US 5556749 A<br>& JP 8-503091 A | 32<br>1-31 |

Form PCT/ISA/210 (continuation of second sheet) (July 1998)

23